Europäisches Patentamt

(19) European Patent Office

Office européen des brevets

(11) EP 0 420 153 B1

(12) EUROPEAN PATENT SPECIFICATION

(45) Date of publication and mention
of the grant of the patent:
05.02.1997 Bulletin 1997/06

(51) Int. Cl.$^6$: **B04B 5/04**, C12M 3/02,
C12N 5/02

(21) Application number: 90118402.8

(22) Date of filing: 25.09.1990

(54) **Centrifugal separator, method of separating animal cells from animal cell-containing suspension using said centrifugal separator, and method of culturing animal cells in suspension using said centrifugal separator**

Zentrifugalseparator, Verfahren zur Trennung von Tierzellen aus Tierzellen enthaltender Suspension mittels dieses Separators und Verfahren zur Züchtung von Tierzellen in Suspension mittels dieses Separators

Séparateur centrifuge, procédé de séparation de cellules animales à partir d'une suspension de cellules animales au moyen dudit séparateur centrifuge et procédé de culture de cellules animales en suspension au moyen dudit séparateur centrifuge

(84) Designated Contracting States:
CH DE FR GB LI

(30) Priority: 27.09.1989 JP 249269/89
24.04.1990 JP 106444/90

(43) Date of publication of application:
03.04.1991 Bulletin 1991/14

(60) Divisional application: 91117364.9

(73) Proprietor: TEIJIN LIMITED
Osaka (JP)

(72) Inventors:
• Tokashiki, Michiyuki
Hachioji-shi, Tokyo (JP)
• Hamamoto, Kimihiko
Hino-shi, Tokyo (JP)
• Ishimaru, Kenji
Iwakuni-shi, Yamaguchi-ken (JP)

(74) Representative: Kraus, Walter, Dr. et al
Patentanwälte Kraus, Weisert & Partner
Thomas-Wimmer-Ring 15
80539 München (DE)

(56) References cited:
EP-A- 0 229 289          GB-A- 873 494

• PATENT ABSTRACTS OF JAPAN, vol. 13, no. 42
(C-564)[3390], 30th January 1989; & JP-A-63 240
783 (TEIJIN LTD) 06-10-1988

**Description**

DETAILED DESCRIPTION OF THE INVENTION

FIELD OF INDUSTRIAL UTILIZATION

This invention relates to a centrifugal separator, a method of separating animal cells and an aqueous solution from a living animal cell-containing aqueous suspension using said centrifugal separator, and a method of culturing animal cells in suspension using said centrifugal separator. More particularly, this invention relates to a centrifugal separator suited for efficiently separating animal cells from an animal cell-containing suspension, and its use.

PRIOR ART

To prevent the growth of cells from stopping at a relatively low cell density and to culture the cells in large quantities at a high density in suspension, there has been generally proposed a so-called perfusion method comprising culturing cells while supplying a fresh culture fluid to a culture tank and meanwhile discharging the spent culture fluid containing a growth-inhibitory substance out of a culture tank (Annual Reports on Fermentation Processes, vol. 6).

In culturing the animal cells by this method, it is important to efficiently separate the spent culture fluid from living cells in the suspension, discharge the spent culture fluid out of the culture tank and maintain the growth environment for the cells in the culture tank under optimum conditions. Various filters or other systems have been proposed for the separation of living cells and the spent culture fluid. None of them, however, have proved to be entirely satisfactory as an industrial device for culturing cells in large quantities because of blockage of the filters or complexity of the structure of the device.

In Japanese Laid-open Patent Application No. 252558/1988, an apparatus for separating animal cells and a method of culturing cells by a perfusion system suited for culturing cells in large quantities at a high density are proposed by the present inventors. Said proposal comprises a centrifugal separating device and a separating system for continuously or intermittently separating living cells and a spent culture fluid from a suspension culture fluid utilizing a centrifugal force. However, there is a fear of damage to cells owing to a pressure difference in the centrifugal separating device occurring when the cells pass through the liquid of a high density used in withdrawing the cells, and this proposal cannot be said to apply to all kinds of cells.

Moreover, since only part of centrifugal separating device can be utilized in the above proposal, the volumetric efficiency is low and the device is therefore not suited to separate large quantities of animal cells within a fixed period of time.

On the other hand, the separation of the animal cells from the animal cell-containing suspension is widely utilized in not only the aforesaid suspension culture but also the separation of cells from blood of mammals, and is therefore an important technology.

Furthermore, GB-A-873 494 discloses a centrifugal separator comprising a centrifugal space consisting of the interior of a peripheral hose of circular cross-section wound in several windings around a conical rotor and having a feed opening for liquid fluid at the central axis of the rotor and a discharge opening for the liquid fluid near the farthest position from the central axis of the rotor. Within that helical hose the liquid forms a corresponding helical continuous flow defined by that hose.

In this centrifugal separator according to GB-A-873 494 the treating amount is relatively small when animal cells are to be separated from culture liquid. Furthermore, pressure loss is very high, i.e. the pump pressure must be raised correspondingly in feeding a culture liquid into that known centrifugal separator. This is disadvantageous from the viewpoint of planning and moreover, there occurs necessarily the problem that the enhanced static pressure causes increased damage of the animal cells. Therefore, the above centrifugal separator of GB-A-873 494 is unsuitable as an apparatus for treating large amounts of culture liquid on an industrial scale.

Finally, from EP-A-0 229 289 and JP-A-63-240 783 (Derwent publication) there is known a method to culture large quantity of animal cells in high density, by separating living cells and culture liquid from a suspension culture liquid with a centrifugal separator in the presence of a specific liquid carrier and returning the separated living cells together with the liquid carrier to a culture tank. In this method a suspension culture liquid containing animal cells is taken out of a culture tank for culturing animal cells in suspended state and is supplied to a centrifugal separator. The centrifugal separator is supplied with a liquid carrier (e.g. a perfluorocarbon) which is essentially immiscible with water, has higher density than water and does not essentially inhibit the growth of the animal cell. The animal cells separated from the culture liquid is discharged together with the liquid carrier and returned to the culture tank. The centrifugal separator used in the above process is provided with a feeding port of the suspension culture liquid, a precipitation surface formed by animal cells moving with centrifugal force, a collection part to collect the cells, a discharging port of the animal cells collected in the collection part and a discharging port of the culture mother liquor. The operation is carried out under a condition to satisfy the following relationships:

(1) $\Theta \leq 300$,

(2) $\overline{Z} \times \Theta \leq 3 \times 10^4$,

(3) $Q/S.\overline{Z} \leq 0.3$, and

(4) $5 \leq \overline{Z} \leq 2000$

wherein

$\Theta$ is an average residence time (minutes) of the animal cells in the centrifugal separator (S),

$\overline{Z}$ is a centrifuging effect,

Q is the amoung (ml/min.) of the suspension culture fluid supplied to the centrifugal separator (S) per unit time, and

S is the sedimentation area (cm$^2$) when the centrifugal force is acting.

OBJECTS OF THE INVENTION

A first object of this invention is to provide a centrifugal separator which can be used to separate animal cells as they are living substantially without any damage from an animal cell-containing suspension.

A second object of this invention is to provide a centrifugal separator which can be used to separate the animal cells and has a high volumic efficiency.

A third object of this invention is to provide a method of driving and operating a centrifugal separator for separating animal cells from the suspension.

A fourth object of this invention is to provide an industrially advantageous method of culturing animal cells in suspension using the centrifugal separator.

A fifth object of this invention is to provide a method of culturing animal cells in suspension at a high density using the centrifugal separator for obtaining useful active proteins produced from the animal cells.

The other objects of this invention will be clarified from the following description.

According to the study of the present inventors, the objects and advantages of this invention are found to be achieved by a centrifugal separator as defined in claim 1, a method of separating animal cells as defined in claim 8, and a method of culturing animal cells as defined in claim 13.

Further developments of the invention are defined in the other claims.

BRIEF DESCRIPTION OF DRAWINGS

Figure 1 is a rough view of a series of devices suitable for practicing a method of culturing animal cells in suspension in accordance with this invention.

Figures 2 and 3(1) are rough perspective views of a rotor of a centrifugal separator in this invention. Figure 3(2) is a plan view of the rotor of the centrifugal separator in Figure 3(1). Figure 3(3) is a plan view of a rotor of another centrifugal separator in this invention.

Figure 4 is a perspective view showing a structure of a rotor of a centrifugal separator that does not belong to this invention.

The centrifugal separator of this invention will be hereinafter described in detail by referring to the drawings.

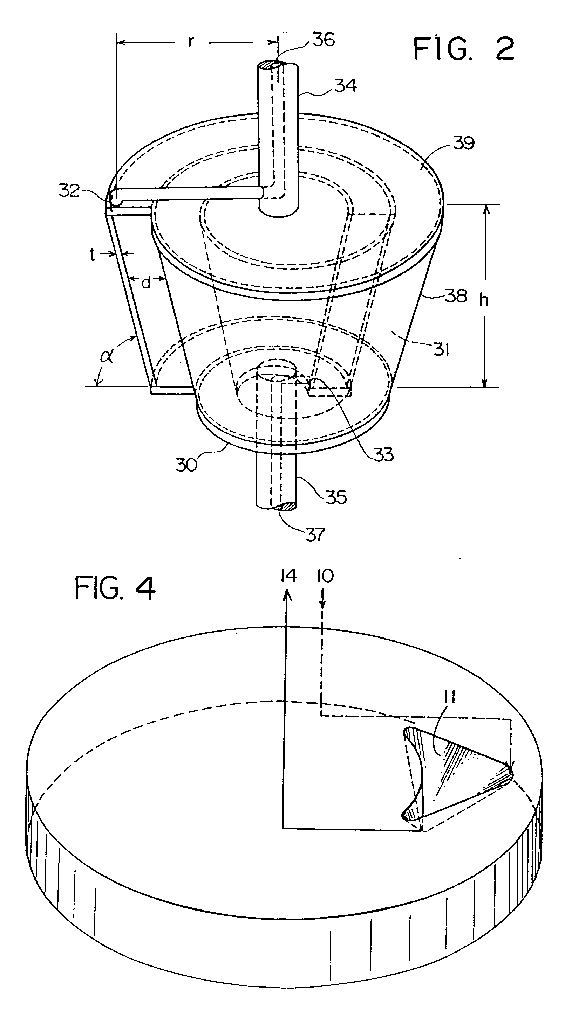

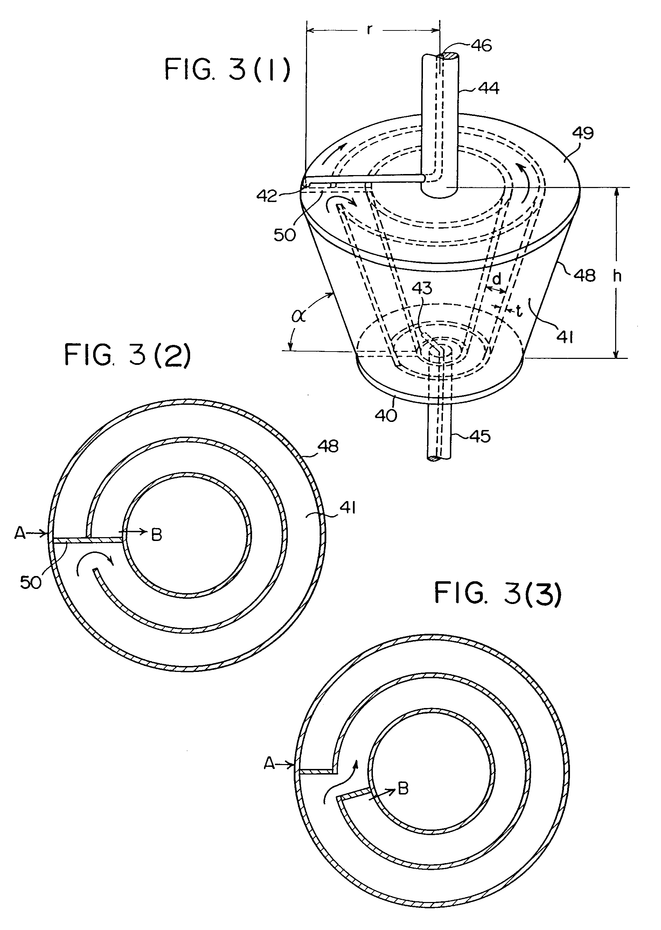

Figure 2 is a partially perspective rough view of a centrifugal separator of this invention. In said Figure 2, the peripheral slot is characterized in that it exists to form a helical continuous flow toward the central axis of the rotor substantially without changing an angle. That is, in Figure 2, the peripheral slot consists of an equidistantly convoluted plate 38 having an inclination angle ($\alpha$) and flat plates 30, 39 for closing. Reference numeral 32 is a feed opening of a liquid fluid such as a suspension culture fluid, which opening is connected with a rotary shaft 34. Reference numeral 33 is a discharge opening of the separated liquid fluid, which opening is connected with the rotary shaft 35. Reference numerals 36 and 37 are nozzles for feeding into, and discharging from, the rotor body. The angle ($\alpha$) between the slot surface and the centrifugal direction in Figure 2 is 30 to 80°, and the depth (d) of the helical peripheral slot is usually 2 to 10 mm. The structure of the peripheral slot in Figure 2 may be a structure shown in Figure 3(3).

Figure 3(1) shows a partially perspective rough view of another centrifugal separator of this invention. In said Figure 3(1), a peripheral slot is characterized in that it exists to form an inversely helical continuous flow toward the central axis of the rotor substantially without changing the angle. Namely, a centrifugal (direction perpendicular to the central axis) sectional plan view is shown in Figure 3(2). In said Figure 3(1), the peripheral slot consists of an equidistantly convoluted plate 48 having an inclination angle ($\alpha$), a partition plate 50 and flat plates 40, 49 for closing. Reference numeral 42 is a feed opening of a liquid fluid such as a suspension culture fluid, which opening is connected with a rotary shaft 44. The feed opening 42 is indicated at A in Figure 3(2). Reference numeral 43 is a discharge opening of the separated liquid fluid, which opening is connected with a rotary shaft 45. The discharge opening 43 is indicated at B in Figure 3(2). From Figures 3(1) and 3(2), it follows that in this centrifugal separator, the peripheral slot is formed such that the liquid fluid fed from the feed opening 42 (or A) forms a 360° (nearly one round) continuous flow toward the central axis of the rotor along the rotating direction without changing the angle, the flow is inverted toward the inside from the partition plate located near the feed opening to form again a 360° (nearly one round) continuous flow in the opposite direction to the rotating direction, and the liquid fluid is thereby discharged from the discharge opening 43 (or B). For simplicity of explanation, a structure of a double peripheral slot which means one inversion of a liquid fluid flow is shown in Figures 3(1) and 3(2). This invention is however not limited thereto, and a structure with two to five inversions of the liquid fluid flow is also available. Reference numerals 46 and 47 are nozzles for feeding into, and discharging from, the rotor body. In Figure 3(1), the angle ($\alpha$) between the slot surface and the centrifuging direction is 30 to 80°, and the depth (d) of the peripheral slot is 2 to 10 mm.

As explained in Figures 2, 3, 3(1), 3(2) and 3(3), the centrifugal separator of this invention is characterized in that a centrifuging space consists of a peripheral slot having an outside peripheral wall inclining toward a central axis of a rotor at an inclination angle of 30 to 80° in a direction perpendicular to the central axis of the rotor, the outside peripheral wall of the peripheral slot exists within an angular range of not less than 360° around the central axis of the rotor, the peripheral slot has such a space that the liquid fluid forms a continuous flow, and said peripheral slot has a feed opening or a discharge opening of the liquid fluid at, or near, the farthest position from the central axis of the rotor, and a feed opening or a discharge opening of the liquid flow at, or near, the nearest position from the central axis of the rotor.

It will be explained hereinafter that animal cells are separated from an animal cell-containing suspension culture fluid by the aforesaid centrifugal separator.

A centrifuging space for receiving the suspension culture fluid consists of a peripheral slot provided in a rotor. The peripheral slot has an outside peripheral wall inclining toward the central axis of the rotor at an inclination angle of 30 to 80°, preferably 35 to 75° in a direction perpendicular to the central axis of the rotor, whereby the separated living animal cells can smoothly be moved on the outside peripheral wall in a direction away from the central axis of the rotor, making it possible to prevent them from being diffused again by the liquid flow.

Moreover, even in separating the accumulated animal cells by pushing them out with a liquid carrier to be described later, the animal cells liable to remain in the peripheral slot, especially, on its inside peripheral wall can completely be withdrawn from inside the peripheral slot because of the inclining outside peripheral wall.

The angle of the outside peripheral wall may be the same throughout, or a combination of two or more angles within the above angular range.

Meanwhile, the inside peripheral wall of the peripheral slot, unlike the outside peripheral wall, does not necessarily have to incline toward the central axis of the rotor. For example, the inside peripheral wall may be parallel, or incline, to the central axis of the rotor. The inside peripheral wall, preferably inclines toward the central axis of the rotor, like the outside peripheral wall, at an inclination angle of 30 to 80°, preferably 35 to 75° in a direction perpendicular to the central axis of the rotor. The inside peripheral wall can be helical to the central axis of the rotor in a phantom plane across the central axis of the rotor.

The peripheral slot exists within a length of the outside wall corresponding to an angular range of not less than 360° around the central axis of the rotor. The desirous length of the outside wall varies with the diameter of the rotor. It is usually at least 720° but at most 3600°, most preferably at least 1080° but at most 2520° around the central axis of the rotor. That is, the peripheral slot can be helical around the central axis of the rotor within the same phantom plane, i.e. without changing the angle toward the central axis of the rotor (e.g. Figure 2). Alternatively, it can be helical, inclining around the central axis of the rotor at a certain inclination angle toward the central axis of the rotor.

The length of the peripheral slot can be increased, as stated above, by providing the peripheral slot having a length corresponding to an angular range of not less than 360° around the central axis of the rotor. Consequently, the separation of the living animal cells can be carried out slowly, smoothly and efficiently. The peripheral slot may be provided therein with a separation plate for aiding in easy and accurate separation of the animal cells by shortening the sedimentation distance. Such separating plate is known per se in the art.

The centrifugal separator is provided in the centrifuging space, i.e. the peripheral slot with a feed opening for feeding the suspension culture fluid and a discarge opening for withdrawing the mother liquor separated from the animal cells during operation of the centrifugal separator, i.e. during rotation of the rotor. The feed opening is formed at, or near, the farthest position from the central axis of the rotor of the peripheral slot; the discharge opening is formed at, or near, the nearest position from the central axis of the rotor of the peripheral slot.

The feed or discharge opening of the liquid fluid formed at, or near, the nearest position from the central axis of the rotor is desirably situated in at least 1/3 of the radius of the rotor from the central axis of the rotor.

The centrifugal separator of this invention is thus provided in the space with the peripheral slot, and said peripheral slot has the outside peripheral wall inclining at the inclination angle toward the central axis of the rotor and is of a structure to form a long continuous flow. The centrifugal separator is therefore quite suited to treat an animal cell-containing suspension and separate the animal cell therefrom. That is, because the substantial area of the peripheral slot can be taken in a quite wide range with respect to the centrifugal separator of the same scale and the laminar flow be formed in the peripheral slot for smooth flow of the suspension, the damage of the animal cells can be extremely minimized.

The centrifugal separator of this invention can thus be said to have such a structure that the substantial area of the peripheral slot for separation is wide and the smooth flow is formed. In other words, the centrifugal separator of this invention has the quite excellent structure to treat the suspension containing animal cells susceptible to damage by exertion of a pressure or physical operation in large quantities per unit time and unit volume (substantial volume of the centrifugal separator) and to separate the living animal cells.

Moreover, according to the study of the present inventors, there are provided [I] a method of separating animal cells and an aqueous solution from a living animal cell-containing suspension using the centrifugal separator of this invention, and [II] a method of culturing animal cells in suspension.

[I] Method of separating animal cells and an aqueous solution from a living animal cell-containing suspension

A method of separating animal cells and an aqueous solution from a living animal cell-containing aqueous suspension, which comprises

(A) feeding a living animal cell-containing aqueous suspension from a feed opening of a liquid fluid in (iv) in the rotating centrifugal separator of this invention for a certain period of time, meanwhile withdrawing the aqueous solution separated from the living animal cells from a discharge opening of the liquid fluid in (v), and accumulating the animal cells in the space of the centrifugal separator,
(B) then feeding a liquid carrier immiscible with water, having a higher density than the animal cells and the aqueous solution and not inhibiting the growth of the animal cells from the feed opening of the liquid fluid in (iv) in the rotating centrifugal separator, withdrawing the living animal cells together with the remaining aqueous solution by pushing them out with the liquid carrier from the discharge opening of the liquid fluid in (v) to thereby obtain the living animal cells, and
(C) further feeding a fresh aqueous solution from the feed opening of the liquid fluid in (v) in the rotating centrifugal separator, and withdrawing the liquid carrier from the discharge opening of the liquid fluid in (iv) to separate the liquid carrier.

[II] Method of culturing animal cells in suspension

A method of culturing animals in suspension, which comprises,

(a) culturing living animal cells in suspension in a culture tank,
(b) withdrawing a portion of the living animal cell-containing suspension culture fluid from the culture tank,
(c) feeding the withdrawn suspension culture fluid from the feed opening of the liquid fluid in (iv) in the rotating centrifugal separator of this invention for a certain period of time, meanwhile withdrawing the culture fluid separated from the living cells via the discharge opening of the liquid fluid in (v), and accumulating the animal cells in the space of the centrifugal separator,
(d) then feeding a liquid carrier immiscible with water, having a higher density than the animal cells and the culture fluid and not inhibiting the growth of the animal cells from the feed opening of the liquid fluid in (iv) in the centrifugal separator, and withdrawing the living animal cells by pushing them out together with the culture fluid from the discharge opening of the liquid fluid in (v),
(e) returning at least a portion of the withdrawn animal cells to the culture tank for step (a), and
(f) further feeding a fresh culture fluid or the culture fluid withdrawn in step (c) from the feed opening of the liquid fluid in (v) in the centrifugal separator, and withdrawing the liquid carrier from the discharge opening of the liquid fluid in (iv) to recover the liquid carrier from the centrifugal separator by separation.

In the separating method [I], the "living animal cell-containing aqueous suspension" being separated can be any solution containing the animal cells in suspension, and its origin is not limited in particular. It may be a suspension withdrawn from a culture tank in suspension culture of animal cells, or body fluids of mammals, especially blood.

The operation of the culturing method [II] in this invention will be explained hereinafter. Said method is not substantially different from the separating method [I]. That is, steps (c), (d) and (f) in the method [II] correspond to steps (A), (B) and (C) in the method [I].

The method [II] of culturing animal cells in accordance with this invention is applicable to the culturing of animal cells in suspension (suspension culture). The suspension culture denotes a method whereby animal cells are cultured while they are suspended in an aqueous medium.

The animal cells to which the culturing method of this invention can be applied are those which can grow or proliferate in suspension. They include not only natural animal cells, but also cells modified artificially or by gene manipulation such as hybridoma cells. Or they may be cells derived from lymphatic cells which produce lymphokine, diploid cells producing biologically active substances such as interferon (IFN), or cells producing various monoclonal antibodies.

The present invention is especially suitable for obtaining monoclonal antibodies at high densities by culturing monoclonal antibody-producing cells.

In step (A) of the method of this invention, the animal cells are cultured in suspension in a culture tank. The culture medium used in the suspension culture is an aqueous medium consisting substantially of water. The aqueous medium contains various additives ordinarily used in the culturing of animal cells, for example inorganic salts, vitamins, coenzymes, glucose, amino acids, antibiotics, and growth promoting factors.

Serum may be added to the culture fluid. But a serum-free culture medium may also be used as the culture fluid. The use of the serum-free culture medium is economically advantageous, and therefore desirable.

The culture tank that can be used in this invention at least comprises an opening for feeding animal cells, an opening for introducing a fresh culture medium, a tube for introduction of air, an agitator, and a conduit for withdrawing the suspension culture fluid. Such a culture tank may be an ordinary culture tank.

In step (b), a portion of the suspension culture fluid containing animal cells is withdrawn from the culture tank, for example through a culture fluid withdrawal conduit. The withdrawal of the suspension culture fluid can be carried out continuously or intermittently. Desirably, a fresh supply of the makeup culture medium is introduced into the culture tank continuously or intermittently in an amount corresponding to the amount of the withdrawn suspension culture fluid containing animal cells. As a result, the concentration of substances which inhibit (adversely affect) the growth of the animal cells and consist mainly of metabolites of the animal cells in the suspension culture fluid within the culture tank can be always maintained at a considerably low level. Thus, the density of the grown animal cells in the suspension culture fluid can be increased.

The amount of useful metabolites produced with the growth of the animal cells can be increased by an amount corresponding to the increased density of the grown animal cells.

By maintaining the concentration of the substances which inhibit the growth of the animal cells at a low level with a sufficient control, the culturing can be continued while maintaining an increased density of the grown animal cells over an extended period of time, and the useful metabolites can be produced in increased amounts.

The above culturing method is characterized by accumulating the living animal cells in the centrifuging space of the centrifugal separator in step (c), and then pushing and withdrawing the accumulated living animal cells together with the mother liquor by the liquid medium immiscible with water, having a higher density than the animal cells and the culture fluid and not hindering the growth of the animal cells in step (d).

In step (c), to accumulate the living animal cells, it is necessary to continuously feed the suspension culture fluid withdrawn in step (b) into the centrifuging space of the centrifugal separator for a certain period of time and to continuously withdraw the mother liquor separated from the living animal cells for a certain period of time. Specifically, since the suspension culture fluid withdrawn in step (b) has a low concentration of the animal cells, it is fed into the centrifuging space until the animal cells are accumulated in a desired amount. The following operating conditions for the centrifugal separator are desirable.

(1) $\theta \leqq 300$,
(2) $\overline{Z} \times \theta \leqq 3 = \times 10^4$,
(3) $Q/S.\overline{Z} \leqq 0.3$, and
(4) $5 \leqq \overline{Z} \leqq 2,000$

   wherein

$\theta$ is the average residence time (minutes) of the animal cells in the centrifugal separator,
$\overline{Z}$ is a centrifuging effect,
$Q$ is the amount (ml/min.) of the suspension culture fluid supplied to the centrifugal separator per unit time, and
$S$ is the sedimentation area ($cm^2$) when the centrifugal force is acting.

The average residence time ($\theta$, minutes) of the animal cells in the centrifugal separator should be limited to not more than 300 minutes. If the residence time exceeds 300 minutes, the survival rate of the animal cells becomes markedly low owing, for example, to the deficiency of oxygen. Preferably, the average residence time ($\theta$) is not more than 150 minutes, especially not more than 60 minutes. Most preferable is not more than 30 minutes but not less than 5 minutes.

For example, in the continuous method comprising continuously feeding the suspension culture fluid into the centrifugal separator and continuously withdrawing the separated animal cells, the average residence time ($\theta$) of the animal cells in the centrifugal separator is obtained as a quotient of the volume ($V$, $cm^3$) of the space in which the animal cells in the fed suspension culture fluid can exist under centrifugal conditions, divided by the rate ($Q_C$, $cm^3/min.$) of withdrawing components containing the separated animal cells from the centrifugal separator.

$\overline{Z}$ is the centrifugal effect in the centrifugal separator, and is represented by $r\omega^2/g$ in which $r$ is the distance (cm) from the axis of rotation, $\omega$ is the rotating angular speed (radians/sec.), and $g$ is the acceleration of gravity ($cm/sec^2$). The centrifuging effect, as it were, represents the magnitude of e centrifugal force exerted on the animal cells, and is therefore determined by the position (distance $r$ from the axis of rotation) of the culture fluid feed opening for feeding the suspension culture fluid to be fed to the centrifugal separator for separation. $\overline{Z}$ is in the range of 5 to 2,000. If it is lower than 5, the separating operation is difficult to perform. If it exceeds 2,000, the centirifugal force on the animal cells is too high and the cells are undesirably ruptured markedly. Advantageously, the centrifuging effect ($\overline{Z}$) should be maintained in the range of 10 to 1,000, especially preferably 20 to 300.

The $\overline{Z} \times \theta$ should be maintained at not more than $3 \times 10^4$. If the centrifuging operation is carried out while the $\overline{Z} \times \theta$ value is above the above-specified range, the survival rate of the animal cells decreases gradually by the compaction of the cells themselves. Especially preferably, the $\overline{Z} \times \theta$ value is not more than $2 \times 10^4$.

S ($cm^2$) is the sedimentation area ($cm^2$) under the action of a centrifugal force. S ($cm^2$) is defined as an effective area involved in separation at the site (r cm from the axis of rotation) of the opening for feeding the suspension culture fluid into the centrifugal separator for separation. When a phantom circle at the site of the feed opening at a distance of r from the axis of rotation does not cross the sedimentation surface, it is obtained as a value of 2 rh by the site (r) of the feed opening and the height (h) of the liquid surface of the suspension culture fluid at the position of the feed opening. When the phantom circle crosses the sedimentation surface, the effective area decreases to the area of that portion which ranges to the site where the phantom circle crosses the sedimentation surface, and is obtained by multiplying 2 rh by the ratio of the angle to the point of crossing. It should be understood that the height (h) of the liquid surface mentioned above is a vertical distance from the deepest position of the separating tank of the centrifugal separator.

The value $S.\overline{Z}$ obtained by multiplying the effective area S and the centrifuging effect $\overline{Z}$ is a parameter that shows the separating ability of the centrifugal separator. In the method of this invention, a value obtained by dividing the amount Q (ml/min.) of the suspension culture fluid supplied to the centrifugal separator per unit time by this parameter, i.e. $Q/S.\overline{Z}$, should be limited to not more than 0.3, preferably not more than 0.2, especially preferably not more than 0.1.

By ensuring the operating conditions (1) to (4), step (C) of the method of this invention makes it possible to separate living animal cells efficiently at a very high survival rate from the suspension culture fluid.

The living animal cells separated by the centrifugal separator and accumulated in the centrifuging space is then, in step (d) according to the above method of this invention, pushed out from the discharge opening together with the mother liquor by feeding a liquid carrier immiscible with water, having a higher density than the animal cells and the culture fluid and not inhibiting the growth of the animal cells to the centrifugal separator in operation from the feed opening instead of the suspension culture fluid.

Perfluorocarbons, for example, can be suitably used as the liquid carrier. Advantageously, the fluorocarbons are liquid at room temperatures, and commercially available fluorocarbons can be widely utilized. For example, fluorocarbons used as heat media or an electrically insulating material, and various fluorocarbons used as artificial blood may be used. Specific examples include perfluoroalkanes having at least 8 carbon atoms, perfluorocycloalkanes (such as perfluorodecalin, perfluoromethyldecalin, and perfluoroalkylcyclohexane, and perfluoroalkylcyclohexanes having 3 to 5 carbon atoms in the alkyl moiety), perfluoroalkyltetrahydrofurans having 5 to 7 carbon atoms in the alkyl moiety, perfluoroalkyltetrahydropyrans having 4 to 6 carbon atoms in the alkyl moiety, and perfluoroadamantanes (such as perfluoroadamantane, perfluoromethyladamantane, perfluorodimethyladamantane, perfluoromethylethyladamantane and perfluorodiethyladamantane). These perfluorocarbons may contain various groups, for example a tertiary amino group. They may be used either singly or in combination.

The combination of steps (c) and (d) enables the animal cells to be separated while they are alive. Moreover, since the centrifuging space incessantly undergoes washing with the liquid carrier, the centrifuging space can always be maintained in an environment suited for the growth of the living animal cells and for an industrially advantageous continuous operation.

At least a portion of the living animal cells having the ability to proliferate which have been withdrawn in step (d) are then, in step (e) according to the culturing method of this invention, returned to the culture tank for step (a).

Moreover, in this invention, in step (f), the fresh culture fluid or the culture fluid withdrawn in step (c) is fed from the feed opening of the liquid fluid in (v) of the rotating centrifugal separator, and the liquid carrier is withdrawn from the discharge opening of the liquid fluid in (iv) to recover it from the centrifugal separator by separation.

The culturing method of this invention is an industrially profitable method that exhibits the aforesaid advantages by repeating steps (b) to (f).

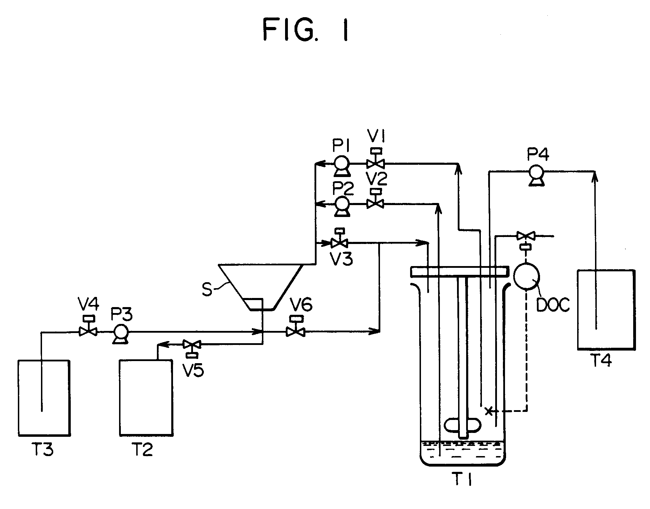

A series of suitable devices for practicing the culturing method of this invention will be explained hereinafter by referring to Figure 1.

Figure 1 is an outline view of an apparaatus suitable for practicing the culturing method of this invention.

In Figure 1, an animal cell-containing suspension culture fluid withdrawn from a culture tank T1 by means of a pump P1 is continuously fed in a centrifugal separator S from a feed opening in (iv) provided at a site under a higher centrifugal force. By the centrifugal force, the animal cells are separated from the suspension fluid, and the mother liquor separated from the animal cells is withdrawn continuously and aseptically from a discharge opening in (v) provided at a site under a lower centrifugal force. On that occasion, it is advisable that the suspension fluid flows in laminar form through the peripheral slot. Thereafter, from the bottom portion of the culture tank T1, a liquid carrier which has no affinity for either the cells or the culture fluid and has a higher density than these is withdrawn by a pump P2, and fed in a fixed amount from the feed opening in (iv) provided at that site of the centrifugal separator S which is under a higher centrifugal force. As a result, the culture fluid containing the separated animal cells in the peripheral slot is aseptically withdrawn from the discharge opening in (v) of the centrifugal separator which is at a site under a lower centrifugal force, and returned to the culture tank T1. Thereafter, a cell-free medium is withdrawn from the tank T3 via the pump P3, and fed from the discharge opening in (v) of the centrifugal separator S, whereby the liquid carrier having a higher density in the centrifugal separator is again returned to the culture tank T1 and the inside of the peripheral slot is replaced by the culture medium. A tank T4 contains a cell-free fresh culture medium which is continuously or intermittently fed into the tank T1 by means of a pump P4 so that the liquid level in the culture tank T1 becomes constant.

By performing the above operation continuously or intermittently without stopping the centrifugal separator, the animal cells can efficiently be separated at a very high survival rate. Needless to say, valves V1 to V6 require an opening-closing operation suitable for the above operation.

As is apparent from the foregoing explanation, the culturing method of this invention is said to be a method which employs the centrifugal separator of the aforesaid structure and uses the liquid carrier immiscible with water, having a higher density than the animal cells and the aqueous solution and not inhibiting the growth of the animal cells, to thereby exhibit the functions thereof most effectively.

That is, in the culturing method of this invention shown in Figure 1, the liquid carrier in the bottom portion of the culture tank is introduced into the centrifugal separator S via the pump P2, the living animal cells accumulated in the centrifugal separator S are thereby pushed out and returned to the culture tank together with the cells. By performing the operation while driving the centrifugal separator, the liquid carrier stays in the centrifugal separator, and the cells which have so far resided therein are spontaneously discharged owing to difference in specific gravity. On this occasion, part or the whole of the liquid carrier is sometimes introduced into the culture tank T1, and therefore the liquid carrier phase is formed at the bottom portion of the culture tank. It will be seen that the above recycling use of the liquid carrier phase formed in the bottom portion of the culture tank is quite advantageous to industrial-scale culturing.

Since the cells do not pass through the liquid carrier having a higher specific gravity by this operation, the exertion of the unnecessary pressure on the cells is avoidable, and damage to the cells is preventable.

According to this invention, the time during which the animal cells pass through the liquid carrier can be reduced to zero or to a very short one. The damage to the animal cells is little if the pressure substantially exerted on the animal cells is 0.2 kgf/cm$^2$ or less. The animal cells are therefore allowed to pass through a liquid carrier having a pressure within this pressure range.

Moreover, by operating this apparatus as described above, the concentration of substances which inhibit the growth of the animal cells accumulated successively within the culture tank as the culturing proceeds can be maintained at low levels.

The following Examples and Comparative Example illustrate this invention more specifically.

EXAMPLE 1

(1) Culture device

A culture system having a cell separating unit of the type shown in Figure 1 was used. The culture tank T1 was a stirred culture tank of stainless steel having a total capacity of 70 liters and designed to receive 40 liters as a net volume of the culture medium.

Into the bottom portion of the culture tank, 6 l of a perfluorocarbon (FLUORINERT® FC-40®, a product of 3M, U. S. A.) was put.

The centrifugal separator was one equipped with a rotor having a sedimentation area (S) of 130 cm$^2$, an effective volume (V) of 230 ml and a rotation radius (r) of 13 cm, as shown in Figure 1.

Pumps P1, P2 and P3 were peristaltic pumps.

(2) Culture medium

A 2:1:1 mixture of RPMI 1640 medium, HAM 12 medium and Dulbecco' modified Eagle medium (to be referred to as RDF) was used as a base medium.

A medium obtained by adding 9 micrograms/ml of insulin, 10 micrograms/ml of transferrin, 10 micrograms/ml of ethanolamine and 2 x 10$^{-6}$ mole/liter of selenous acid to a base medium was used.

(3) Method and results of culturing

The culture system was sterilized in advance by autoclaving. Then, 40 liters of the culture medium sterilized by filtration was fed into the culture tank, and mouse-human hybridoma X87 cells obtained by fusing mouse myeloma P3U1 cells and human B cells were seeded so that the cell density became 8.1 x 10$^5$ cells/ml. (The maximum density of living cells in the quiescent culture of X87 cells reached 1.1 x 10$^6$ cells/ml.) These hybridoma cells produced IgG. Oxygen gas was introduced into the culture tank through a blow nozzle while the concentration of dissolved oxygen in the culture medium was automatically controlled to 3 ppm. The culture fluid in the culture tank was maintained at 37 °C. A marine-type stirring vane was attached to the cultivation tank, and operated at a stirring speed of 30 rpm.

Perfusion culture using the centrifugal separator was started just after inoculation. Specifically, the centrifugal separator charged with the filtration-sterilized culture fluid was driven, and the rotation speed of the centrifugal separator was adjusted so that the centrifuging effect became 80G.

Then, 1,500 ml of the culture fluid was sent to the centrifugal separator over 10 minutes at a rate of 150 ml/min. The mother liquor separated from the cells by the centrifugal separator flowed into the reservoir tank T2 for the spent culture medium and was stored. The density of the viable cell therein was about $3 \times 10^4$ cells/ml and the total cell density was $2.5 \times 10^5$ cells/ml.

After the foregoing operation was terminated, pump P-2 was driven to send 300 ml of the perfluorocarbon at the bottom of the culture tank T1 at a rate of 150 ml/min. By this operation, all the cells residing in the centrifugal rotor were discharged from the centrifugal separator together with the perfluorocarbon, and returned to the culture tank T1.

Thereafter, pump P2 was stopped, and pump P3 was driven to send the fresh medium in T3 to the centrifugal separator S and return the perfluorocarbon in the centrifugal separator to the culture tank T1.

The culturing was continued by automatically performing this operation once every period of time shown in Table 1. A fresh supply of the culture medium was fed continuously into the culture tank so that the liquid level in the culture tank became constant on an average.

The experimental results are shown in Table 1 together with some of the experimental conditions.

In the above experiment,

$\theta = 6$ min.
$\overline{Z} = 80$.

Therefore,

$\overline{Z} \times \theta = 480$ min.
$Q/S.\overline{Z} = 0.0032$ cm/min.

Table 1

| Culturing time (days) | Time interval for feeding to the centrifuge (min) | Specific perfusion rate (vol/vol/day) | Viable cell density (cells/ml) | Antibody concentration in the culture fluid ($\mu$g/ml) |
|---|---|---|---|---|
| 0 | 45 | 1.0 | $8.1 \times 10^5$ | - |
| 1 | 45 | 1.0 | $1.1 \times 10^6$ | - |
| 2 | 45 | 1.0 | $1.7 \times 10^6$ | 9 |
| 3 | 45 | 1.0 | $3.2 \times 10^6$ | 12 |
| 4 | 30 | 1.5 | $4.8 \times 10^6$ | 14 |
| 5 | 30 | 1.5 | $7.8 \times 10^6$ | 35 |
| 6 | 30 | 1.5 | $8.6 \times 10^6$ | 41 |
| 7 | 30 | 1.5 | $9.6 \times 10^6$ | 47 |
| 8 | 30 | 1.5 | $9.8 \times 10^6$ | 53 |
| 9 | 30 | 1.5 | $9.4 \times 10^6$ | 59 |
| 11 | 30 | 1.5 | $9.0 \times 10^6$ | 51 |
| 12 | 30 | 1.5 | $1.1 \times 10^7$ | 57 |
| 14 | 30 | 1.5 | $1.0 \times 10^7$ | 59 |
| 15 | 30 | 1.5 | $8.4 \times 10^6$ | 61 |
| 17 | 30 | 1.5 | $8.6 \times 10^6$ | 56 |
| 18 | 30 | 1.5 | $9.2 \times 10^6$ | 18 |
| 19 | 30 | 1.5 | $1.1 \times 10^7$ | 58 |
| 20 | 30 | 1.5 | $9.6 \times 10^6$ | 60 |
| 21 | 30 | 1.5 | $9.8 \times 10^6$ | 51 |
| 22 | 30 | 1.5 | $9.0 \times 10^6$ | 57 |
| 23 | 30 | 1.5 | $9.4 \times 10^6$ | 55 |

EXAMPLE 2

(1) Culture device

There was used the same culture device as in Example 1 except a rotor of a centrifugal separator. A rotor with a helical multilayered separation zone having the following characteristics, as shown in Figure 2, was used as the rotor of the centrifugal separator.

r = 13 cm
$\alpha$ = 60°
d = 0.4 cm
t = 0.4 cm
h = 2.9 cm
n*= 3
V = 300 ml
S = 590 cm$^2$

* Number of layers of a sedimentation area

(2) Culture medium

The same culture medium as in Example 1 was used.

(3) Method and results of culturing

The culture system was sterilized in advance by autoclaving. Then, 40 liters of the culture medium sterilized by filtration was fed into the culture tank, and mouse-human hybridoma X87 cells were seeded so that the cell density became $2.1 \times 10^5$ cells/ml. These hybrodoma cells produced IgG. Oxygen gas was introduced into the culture tank through a blow nozzle while the concentration of dissolved oxygen in the culture medium was automatically controlled to 3 ppm. The culture fluid in the culture tank was maintained at 37 °C.

For 3 days after the seeding, the culturing was carried out batchwise. As shown in Table 2, the cell density reached $6.3 \times 10^5$ cells/ml on the third day after the start of culturing. Perfusion culture using the centrifugal separator was started. Specifically, the centrifugal separator charged with the filtration-sterilized culture fluid was driven, and the rotation speed of the centrifugal separator was adjusted so that the centrifuging effect became 100G.

Then, 2,000 ml of the culture fluid was sent to the centrifugal separator over 10 minutes at a rate of 200 ml/min. The mother liquor separated from the cells by the centrifugal separator flowed into the reservoir tank T2 for the spent culture medium and was stored. The density of the viable cell therein was about $2 \times 10^4$ cells/ml.

After the foregoing operation was terminated, pump P-2 was driven to send 400 ml of the perfluorocarbon at the bottom of the culture tank T1 at a rate of 200 ml/min. By this operation, all the cells residing in the centrifugal rotor were discharged from the centrifugal separator together with the perfluorocarbon, and returned to the culture tank T1.

Thereafter, pump P2 was stopped, and pump P3 was driven to send the fresh medium in T3 to the centrifugal separator S and return the perfluorocarbon in the centrifugal separator to the culture tank T1.

The culturing was continued by automatically performing this operation once every period of time shown in Table 2. A fresh supply of the culture medium was fed continuously into the culture tank so that the liquid level in the culture tank became constant on an average.

The experimental results are shown in Table 2 together with some of the experimental conditions.

In the above experiment,

$\theta = 6$ min.
$\overline{Z} = 100$.

Therefore,

$\overline{Z} \times \theta = 600$ min.
$Q/S.\overline{Z} = 0.0034$ cm/min.

Table 2

| Culturing time (days) | Time interval for feeding to the centrifuge (min) | Specific perfusion rate (vol/vol/day) | Viable cell density (cells/ml) | Antibody concentration in the culture fluid ($\mu$g/ml) |
|---|---|---|---|---|
| 0 | - | 0 | $2.1 \times 10^5$ | - |
| 1 | - | 0 | $2.8 \times 10^5$ | - |
| 2 | - | 0 | $5.0 \times 10^5$ | - |
| 3 | - | 1.0 | $6.3 \times 10^5$ | - |
| 6 | 60 | 1.0 | $3.7 \times 10^6$ | 21 |
| 7 | 60 | 1.0 | $4.3 \times 10^6$ | 35 |
| 8 | 60 | 1.0 | $7.2 \times 10^6$ | 48 |
| 9 | 60 | 1.0 | $6.3 \times 10^6$ | 51 |
| 10 | 60 | 1.0 | $7.3 \times 10^6$ | 49 |
| 11 | 60 | 1.0 | $9.0 \times 10^6$ | 61 |
| 13 | 60 | 1.0 | $7.6 \times 10^6$ | 70 |
| 15 | 60 | 1.0 | $8.0 \times 10^6$ | 67 |
| 17 | 60 | 1.0 | $7.3 \times 10^6$ | 72 |
| 20 | 60 | 1.0 | $7.8 \times 10^6$ | 87 |
| 22 | 60 | 1.0 | $7.7 \times 10^6$ | 78 |

EXAMPLE 3

(1) Culture device

A culture system having a cell separating unit of the type shown in Figure 3(1) was used. The culture tank T1 was a stirred culture tank of glass having a total capacity of 95 liters and designed to receive 60 liters as a net volume of the culture medium.

Into the bottom portion of the culture tank, 10 l of a perfluorocarbon was put.

A rotor with a concentrically multilayered separation zone, having the following characteristics, as shown in Figure 3(1), was used as the rotor of the centrifugal separator.

r = 17 cm
$\alpha$ = 45°
d = 0.4 cm
t = 0.4 cm
h = 3 cm
n = 2
V = 230 ml
S = 890 cm$^2$

(2) Culture medium

A 2:1:1 mixture of RPMI 1640 medium, HAM 12 medium and Dulbecco' modified Eagle medium (to be referred to as RDF) was used as a base medium.

A medium obtained by adding 9 micrograms/ml of insulin, 10 micrograms/ml of transferrin, 10 micrograms/ml of ethanolamine and $2 \times 10^{-6}$ mole/liter of selenous acid to a base medium was used.

(3) Method and results of culturing

The culture system was sterilized in advance by autoclaving. Then, 60 liters of the culture medium sterilized by filtration was fed into the culture tank, and mouse-human hybridoma P-8 cells obtained by fusing mouse myeloma P3U1 cells and human B cells were seeded so that the cell density became $1.06 \times 10^6$ cells/ml. (The maximum density of living cells in the quiscent culture of P-8 cells reached $1.3 \times 10^6$ cells/ml.) These hybridoma cells produce IgG. Oxygen gas was introduced into the culture tank through a blow nozzle while the concentration of dissolved oxygen in the culture medium was automatically controlled to 3 ppm. The culture fluid in the culture tank was maintained at 37 °C. A marine-type stirring vane was attached to the cultivation tank, and operated at a stirring speed of 30 rpm.

Perfusion culture using the centrifugal separator was started just after inoculation. Specifically, the centrifugal separator charged with the filtration-sterilized culture fluid was driven, and the rotation speed of the centrifugal separator was adjusted so that the centrifuging effect became 120G.

Then, 2,500 ml of the culture fluid was sent to the centrifugal separator over 10 minutes at a rate of 250 ml/min. The mother liquor separated from the cells by the centrifugal separator flowed into the reservoir tank T2 for the spent culture medium and was stored. The density of the viable cell therein was about $4 \times 10^4$ cells/ml.

After the foregoing operation was terminated, pump P-2 was driven to send 300 ml of the perfluorocarbon at the bottom of the culture tank T1 at a rate of 150 ml/min. By this operation, all the cells residing in the centrifugal rotor were discharged from the centrifugal separator together with the perfluorocarbon, and returned to the culture tank T1.

Thereafter, pump P2 was stopped, and pump P3 was driven to send the fresh medium in T3 to the centrifugal separator S and return the perfluorocarbon in the centrifugal separator to the culture tank T1.

The culturing was continued by automatically performing this operation once every period of time shown in Table 3. A fresh supply of the culture medium was fed continuously into the culture tank so that the liquid level in the culture tank became constant on an average.

The experimental results are shown in Table 3 together with some of the experimental conditions.

In the above experiment,

$\theta$ = 6 min.
$\overline{Z}$ = 120.

Therefore,

$\overline{Z} \times \theta$ = 720 min.
$Q/S.\overline{Z}$ = 0.0023 cm/min.

Table 3

| Culturing time (days) | Time interval for feeding to the centrifuge (min) | Specific perfusion rate (vol/vol/day) | Viable cell density (cells/ml) | Antibody concentration in the culture fluid ($\mu$g/ml) |
|---|---|---|---|---|
| 0 | 55 | 1.0 | $1.06 \times 10^6$ | - |
| 1 | 55 | 1.0 | $2.7 \times 10^6$ | - |
| 2 | 36 | 1.5 | $4.2 \times 10^6$ | - |
| 3 | 27 | 2.0 | $6.3 \times 10^6$ | - |
| 4 | 27 | 2.0 | $6.8 \times 10^6$ | 37 |
| 5 | 27 | 2.0 | $9.1 \times 10^6$ | 42 |
| 7 | 27 | 2.0 | $1.2 \times 10^7$ | 51 |
| 9 | 27 | 2.0 | $1.7 \times 10^7$ | 65 |
| 11 | 27 | 2.0 | $1.8 \times 10^7$ | 76 |
| 12 | 27 | 2.0 | $1.5 \times 10^7$ | 82 |
| 14 | 27 | 2.0 | $1.8 \times 10^7$ | 83 |
| 16 | 27 | 2.0 | $1.7 \times 10^7$ | 75 |
| 18 | 27 | 2.0 | $1.6 \times 10^7$ | 81 |

COMPARATIVE EXAMPLE

(1) Culture device

There was used the same culture device as in Example 1 except a rotor of a centrifugal separator. A rotor having a structure shown in Figure 4 was used as the rotor of the centrifugal separator. The volume of the separation zone was 220 ml which was approximately the same as that of the rotor in Example 1.

(2) Culture medium

The same culture medium as in Example 1 was used.

(3) Method and results of culturing

Mouse-human hybridoma cells were cultivated in the same way as in Example 1. Namely, cells were seeded at a density of $1.3 \times 10^5$ cells/ml and culturing started. For 4 days after the seeding, the culturing was conducted batchwise. Four days later, the density of the cells was $6.8 \times 10^5$ cells/ml. At this time, the centrifugal separator was driven. The centrifugal effect was set at 80G. Separation of the cells and the culture fluid was carried out under the same conditions as in Example 1, and the culturing was conducted at a perfusion rate of 1.0 vol/vol/day. The results are shown in Table 4 together with the conditions. After the start of culturing, the density of the cells in the culture tank started to decrease. The density of the cells in the culture supernatant obtained from the centrifugal separator was measured and found to be nearly the same as that of the cells in the culture tank. This means that most of the cells were separated by the centrifugal separator. With the lapse of 8 days after the start of the culturing, the culturing was terminated.

Table 4

| Culturing time (days) | Time interval for feeding to the centrifuge (min) | Specific perfusion rate (vol/vol/day) | Viable cell density (cells/ml) | Antibody concentration in the culture fluid ($\mu$g/ml) |
|---|---|---|---|---|
| 0 | - | 0 | $1.2 \times 10^5$ | - |
| 1 | - | 0 | $1.9 \times 10^5$ | - |
| 2 | - | 0 | $3.2 \times 10^5$ | - |
| 3 | - | 0 | $5.7 \times 10^5$ | - |
| 4 | - | 1.0 | $6.8 \times 10^5$ | - |
| 5 | | 1.0 | $5.3 \times 10^5$ | - |
| 6 | | 1.0 | $4.6 \times 10^5$ | - |
| 7 | | 1.0 | $4.0 \times 10^5$ | - |
| 8 | | 1.0 | $3.4 \times 10^5$ | - |

**Claims**

1.  A centrifugal separator (S), comprising a rotor, is characterized in that

    -   said rotor is radially formed of a conical, spiral wall (38) providing for a spiral conical slot to serve as the liquid fluid containing space,
    -   the said conical wall (38) enclosing an inclination angle (90° - $\alpha$) with the central axis of the rotor,
    -   said rotor comprising a first opening (32) for the liquid fluid substantially at the position farthest from the central axis of said rotor,
    -   and further comprising a second opening (33) for the liquid fluid substantially at the position nearest to the central axis of said rotor,
    -   where there is only one slot over the height of the said rotor.

2.  A centrifugal separator (S), comprising a rotor, is characterized in that

    -   said rotor is radially formed of a conical, spiral wall (48) providing for a spiral conical slot to serve as the liquid fluid containing space,
    -   the said conical wall (48) enclosing an inclination angle (90° - $\alpha$) with the central axis of the rotor,
    -   said rotor comprising a first opening (42) for the liquid fluid substantially at the position farthest from the central axis of said rotor,
    -   and further comprising a second opening (43) for the liquid fluid substantially at the position nearest to the central axis of said rotor,
    -   where there are coaxial conical slots with a communicating opening that extends over the height of the slots where they are adjacent.

3.  The centrifugal separator (S) of claim 1 or 2, characterized in that the inclination angle (90°-$\alpha$) is in the range of 10° to 60°.

4.  The centrifugal separator (S) of claim 2, characterized in that the continuous flow of the liquid fluid in the liquid fluid space is conducted in left or right hand direction or subsequently an alternation of both.

5.  The centrifugal separator (S) of any one of claims 1 to 4, characterized in that the upper limit of the liquid fluid space is present in one plane.

6.  The centrifugal separator (S) of any one of claims 1 to 5, characterized in that the lower limit of the liquid fluid space is present in one plane.

7. The centrifugal separator (S) of any one of claims 1 to 6, characterized in that the liquid fluid space has a depth (d) of 2 to 10 mm.

8. A method of separating animal cells and an aqueous solution from a living animal cell-containing aqueous suspension, which is characterized by comprising

    (A) feeding a living animal cell-containing aqueous suspension from the first opening (32, 42, A) for the liquid fluid in the rotating centrifugal separator (S) according to any one of claims 1 to 7, meanwhile withdrawing the aqueous solution separated from the living animal cells via the second opening (33, 43, B) for the liquid fluid, and accumulating the animal cells in the space of the centrifugal separator (S),

    (B) then feeding a liquid carrier immiscible with water, having a higher density than the animal cells and the aqueous solution and not inhibiting the growth of the animal cells from the first opening (32, 42, A) for the liquid fluid in the rotating centrifugal separator (S), withdrawing the living animal cells together with the remaining aqueous solution by pushing them out with the liquid carrier from the second opening (33, 43, B) for the liquid fluid to thereby obtain the living animal cells, and

    (C) further feeding a fresh or spent medium solution from the second opening (33, 43, B) for the liquid fluid in the rotating centrifugal separator (S), and withdrawing the liquid carrier from the first opening (32, 42, A) for the liquid fluid to separate the liquid carrier.

9. The method of claim 8, characterized in that the living animal cell-containing aqueous suspension is an artificially cultured suspension.

10. The method of claim 8, characterized in that the living animal cell-containing aqueous suspension is a blood from a mammal.

11. The method of claim 8, 9 or 10, characterized in that the liquid carrier is a fluorocarbon.

12. The method of any one of claims 8 to 11, characterized in that the centrifugal separator (S) is operated under the following conditions:

    (1) $\Theta \leq 300$,
    (2) $\overline{Z} \times \Theta \leq 3 \times 10^4$,
    (3) $Q/S.\overline{Z} \leq 0.3$, and
    (4) $5 \leq \overline{Z} \leq 2000$

    wherein

    $\Theta$ is an average residence time (minutes) of the animal cells in the centrifugal separator (S),
    $\overline{Z}$ is a centrifuging effect,
    Q is the amount (ml/min.) of the suspension culture fluid supplied to the centrifugal separator (S) per unit time, and
    S is the sedimentation area ($cm^2$) when the centrifugal force is acting.

13. A method of culturing animal cells in suspension, which is characterized by comprising

    (a) culturing living animal cells in suspension in a culture tank (T1),

    (b) withdrawing a portion of the living animal cell-containing suspension culture fluid from the culture tank (T1),

    (c) feeding the withdrawn suspension culture fluid from the first opening (32, 42, A) for the liquid fluid in the rotating centrifugal separator (S) according to any one of claims 1 to 7 for a certain period of time, meanwhile withdrawing the culture fluid separated from the living cells via the second opening (33, 43, B) for the liquid fluid, and accumulating the animal cells in the space of the centrifugal separator (S),

    (d) then feeding a liquid carrier immiscible with water, having a higher density than the animal cells and the culture fluid and not inhibiting the growth of the animal cells from the first opening (32, 42, A) for the liquid fluid in the centrifugal separator (S), and withdrawing the living animal cells by pushing them out together with the culture fluid from the second opening (33, 43, B) for the liquid fluid,

(e) returning at least a portion of the withdrawn animal cells to the culture tank (T1) for step (a), and

(f) further feeding a fresh culture fluid or the culture fluid withdrawn in step (c) from the second opening (33, 43, B) for the liquid fluid in the centrifugal separator (S), and withdrawing the liquid carrier from the first opening (32, 42, A) for the liquid fluid to recover the liquid carrier from the first opening (32, 42, A) for the liquid fluid by separation.

**14.** The method of claim 13, characterized in that steps (b) to (f) are repeated.

**15.** The method of claim 13 or 14, characterized in that the animal cells are hydridoma cells.

**16.** The method of any one of claims 13 to 15, characterized in that the liquid carrier is a fluorocarbon.

**17.** The method of any one of claims 13 to 16, characterized in that the centrifugal separator (S) is operated under the following conditions:

(1) $\Theta \leq 300$,
(2) $\bar{Z} \times \Theta \leq 3 \times 10^4$,
(3) $Q/S.\bar{Z} \leq 0.3$, and
(4) $5 \leq \bar{Z} \leq 2000$

wherein

$\Theta$ is an average residence time (minutes) of the animal cells in the centrifugal separator (S),
$\bar{Z}$ is a centrifuging effect,
Q is the amount (ml/min.) of the suspension culture fluid supplied to the centrifugal separator (S) per unit time, and
S is the sedimentation area ($cm^2$) when the centrifugal force is acting.

**Patentansprüche**

1. Fliehkraft-Trenneinrichtung (S) mit einem Läufer, dadurch **gekennzeichnet,** daß

   - der genannte Läufer radial aus einer konischen, spiraligen Wand (38) gebildet ist, die für einen spiraligen, konischen Schlitz sorgt, der als Raum zur Aufnahme flüssigen Strömungsmittels dient,
   - die genannte konische Wand (38) einen Neigungswinkel (90° - $\alpha$) zur Mittelachse des Läufers einschließt,
   - der genannte Läufer eine erste Öffnung (32) für das flüssige Strömungsmittel im wesentlichen an der Stelle aufweist, die von der Mittelachse des genannten Läufers am weitesten entfernt ist, und
   - dieser ferner eine zweite Öffnung (33) für das flüssige Strömungsmittel im wesentlichen an der Stelle aufweist, die der Mittelachse des genannten Läufers nächstgelegen ist,
   - wobei nur ein einziger Schlitz über die Höhenerstreckung des genannten Läufers vorliegt.

2. Fliehkraft-Trenneinrichtung (S) mit einem Läufer, dadurch **gekennzeichnet,** daß

   - der genannte Läufer radial aus einer konischen, spiraligen Wand (48) gebildet ist, die für einen spiraligen, konischen Schlitz sorgt, der als Raum zur Aufnahme von flüssigem Strömungsmittel dient,
   - die genannte konische Wand (48) einen Neigungswinkel (90° - $\alpha$) mit der Mittelachse des Läufers einschließt,
   - der genannte Läufer eine erste Öffnung (42) für das flüssige Strömungsmittel im wesentlichen an der Stelle aufweist, die von der Mittelachse des genannten Läufers am weitesten entfernt ist, und
   - er eine zweite Öffnung (43) für das flüssige Strömungsmittel aufweist, die im wesentlichen an der Stelle liegt, die der Achse des genannten Läufers nächstgelegen ist,
   - wobei koaxiale, konische Schlitze mit einer Verbindungsöffnung vorliegen, die sich über die Höhenerstreckung der Schlitze dort erstreckt, wo sie nebeneinander liegen.

3. Fliehkraft-Trenneinrichtung (S) nach Anspruch 1 oder 2, dadurch **gekennzeichnet,** daß der Neigungswinkel (90° - $\alpha$) im Bereich von 10° bis 60° liegt.

4. Fliehkraft-Trenneinrichtung (S) nach Anspruch 2, dadurch **gekennzeichnet,** daß der kontinuierliche Fluß des flüssigen Strömungsmittels im Raum für flüssiges Strömungsmittel in einer Richtung nach links oder rechts oder einer aufeinanderfolgenden Abwechslung dieser beiden geleitet wird.

5. Fliehkraft-Trenneinrichtung (S) nach irgendeinem der Ansprüche 1 bis 4, dadurch **gekennzeichnet,** daß die obere Grenze des Raums für flüssiges Strömungsmittel in einer einzigen Ebene vorliegt.

6. Fliehkraft-Trenneinrichtung (S) nach irgendeinem der Ansprüche 1 bis 5, dadurch **gekennzeichnet,** daß die untere Grenze des Raums für flüssiges Strömungsmittel in einer einzigen Ebene vorliegt.

7. Fliehkraft-Trenneinrichtung (S) nach irgendeinem der Ansprüche 1 bis 6, dadurch **gekennzeichnet,** daß der Raum für flüssiges Strömungsmittel eine Tiefe (d) von 2 bis 10 mm aufweist.

8. Verfahren zum Abtrennen von Tierzellen und einer wäßrigen Lösung aus einer lebende Tierzellen enthaltenden, wäßrigen Suspension, **gekennzeichnet** durch die folgenden Merkmale:

   (A) Einspeisen einer lebende Tierzellen enthaltenden, wäßrigen Lösung von der ersten Öffnung (32, 42, A) für das flüssige Strömungsmittel in der rotierenden Fliehkraft-Trenneinrichtung (S) her gemäß irgendeinem der Ansprüche 1 bis 7, währenddessen Abziehen der wäßrigen Lösung, die von den lebenden Tierzellen abgetrennt ist, über die zweite Öffnung (33, 43, B) für das flüssige Strömungsmittel, und Ansammeln der Tierzellen im Raum der Fliehkraft-Trenneinrichtung (S),
   (B) dann Einspeisen eines flüssigen Trägers, der mit Wasser unvermischbar ist, eine höhere Dichte aufweist als die Tierzellen und die wäßrige Lösung und das Wachstum der Tierzellen nicht inhibiert, von der ersten Öffnung (32, 42, A) für das flüssige Strömungsmittel in der rotierenden Fliehkraft-Trenneinrichtung (S) her, und Abziehen der lebenden Tierzellen zusammen mit der verbleibenden wäßrigen Lösung dadurch, daß man sie zusammen mit dem flüssigen Träger aus der zweiten Öffnung (33, 43, B) für das flüssige Strömungsmittel hinausdrückt, um hierdurch die lebenden Tierzellen zu erhalten, und
   (C) weiteres Einspeisen einer frischen oder verbrauchten Lösung des Mediums von der zweiten Öffnung (33, 43, B) für das flüssige Strömungsmittel in der rotierenden Fliehkraft-Trenneinrichtung (S) her, und Abziehen des flüssigen Trägers aus der ersten Öffnung (32, 42, A) für das flüssige Strömungsmittel zum Abtrennen des flüssigen Trägers.

9. Verfahren nach Anspruch 8, dadurch **gekennzeichnet,** daß die lebende Tierzellen enthaltende, wäßrige Suspension eine künstlich gezüchtete Suspension ist.

10. Verfahren nach Anspruch 8, dadurch **gekennzeichnet,** daß die lebende Tierzellen enthaltende, wäßrige Suspension Blut von einem Säugetier ist.

11. Verfahren nach Anspruch 8, 9 oder 10, dadurch **gekennzeichnet,** daß der flüssige Träger ein Fluorkohlenstoff ist.

12. Verfahren nach irgendeinem der Ansprüche 8 bis 11, dadurch **gekennzeichnet,** daß die Fliehkraft-Trenneinrichtung (S) unter den folgenden Bedingungen betrieben wird:

   (1) $\theta \leq 300$,
   (2) $\overline{Z} \times \theta \leq 3 \times 10^4$,
   (3) $Q/S.\overline{Z} \leq 0{,}3$, und
   (4) $5 \leq \overline{Z} \leq 2000$

worin

   $\theta$ die mittlere Verweilzeit (Minuten) der Tierzellen in der Fliehkraft-Trenneinrichtung (S) ist,
   $\overline{Z}$ eine Fliehkraftwirkung ist,
   Q die Menge (ml/min) der eine Suspension bildenden Kulturflüssigkeit ist, die der Fliehkraft-Trenneinrichtung (S) pro Zeiteinheit zugeführt wird, und
   S die Absetzfläche (cm²) ist, wenn die Fliehkraft wirksam ist.

13. Verfahren zum Züchten von Tierzellen in Suspension, **gekennzeichnet** durch die folgenden Merkmale:

   (a) Züchten lebender Tierzellen in Suspension in einem Kulturtank (T1),

18

(b) Abziehen eines Anteils der lebende Tierzellen enthaltenden, in Suspension befindlichen Kulturflüssigkeit aus dem Kulturtank (T1),

(c) Einspeisen der abgezogenen, in Suspension befindlichen Kulturflüssigkeit von der ersten Öffnung (32, 42, A) für das flüssige Strömungsmittel in der rotierenden Fliehkraft-Trenneinrichtung (S) her gemäß irgendeinem der Ansprüche 1 bis 7 für einen bestimmten Zeitraum, währenddessen Abziehen der Kulturflüssigkeit, die von den lebenden Zellen abgetrennt wurde, über die zweite Öffnung (33, 43, B) für das flüssige Strömungsmittel, und Ansammeln der Tierzellen in dem Raum der Fliehkraft-Trenneinrichtung (S),

(d) dann Einspeisen eines flüssigen Trägers, der mit Wasser unvermischbar ist, eine höhere Dichte als die Tierzellen und die Kulturflüssigkeit aufweist und das Wachstum der Tierzellen nicht inhibiert, von der ersten Öffnung (32, 42, A) für das flüssige Strömungsmittel in der Fliehkraft-Trenneinrichtung (S) her, und Abziehen der lebenden Tierzellen dadurch, daß man sie zusammen mit der Kulturflüssigkeit aus der zweiten Öffnung (33, 43, B) für das flüssige Strömungsmittel hinausdrückt,

(e) Rückführen mindestens eines Anteils der abgezogenen Tierzellen zum Kulturtank (T1) für den Schritt (a), und

(f) weiter Einspeisen einer frischen Kulturflüssigkeit oder der Kulturflüssigkeit, die im Schritt (c) abgezogen wurde, von der zweiten Öffnung (33, 43, B) für das flüssige Strömungsmittel in der Fliehkraft-Trenneinrichtung (S) her, und Abziehen des flüssigen Trägers aus der ersten Öffnung (32, 42, A) für das flüssige Strömungsmittel, um den flüssigen Träger aus der ersten Öffnung (32, 42, A) für das flüssige Strömungsmittel durch Abtrennung wiederzugewinnen.

14. Verfahren nach Anspruch 13, dadurch **gekennzeichnet,** daß die Schritte (b) bis (f) wiederholt werden.

15. Verfahren nach Anspruch 13 oder 14, dadurch **gekennzeichnet,** daß die Tierzellen Hybridomzellen sind.

16. Verfahren nach irgendeinem der Ansprüche 13 bis 15, dadurch **gekennzeichnet,** daß der flüssige Träger ein Fluorkohlenstoff ist.

17. Verfahren nach irgendeinem der Ansprüche 13 bis 16, dadurch **gekennzeichnet,** daß die Fliehkraft-Trenneinrichtung (S) unter den folgenden Bedingungen betrieben wird:

(1) $\theta \leq 300$,
(2) $\overline{Z} \times \theta \leq 3 \times 10^4$,
(3) $Q/S.\overline{Z} \leq 0,3$, und
(4) $5 \leq \overline{Z} \leq 2000$

worin

$\theta$ die mittlere Verweilzeit (Minuten) der Tierzellen in der Fliehkraft-Trenneinrichtung (S) ist,
$\overline{Z}$ eine Fliehkraftwirkung ist,
Q die Menge (ml/min) der eine Suspension bildenden Kulturflüssigkeit ist, die der Fliehkraft-Trenneinrichtung (S) pro Zeiteinheit zugeführt wird, und
S die Absetzfläche ($cm^2$) ist, wenn die Fliehkraft wirksam ist.

**Revendications**

1. Séparateur centrifuge (S), comprenant un rotor, caractérisé en ce que

- ledit rotor est formé radialement d'une paroi spirale conique (38) servant de fente conique spirale qui sert de l'espace contenant le liquide,
- ladite paroi conique (38) comprenant un angle d'inclinaison (90° - $\alpha$) à l'axe central du rotor,
- ledit rotor comprenant un premier orifice (32) pour le liquide essentiellement au point le plus éloigné de l'axe central dudit rotor,
- et ensuite comprenant un deuxième orifice (33) pour le liquide essentiellement au point le plus proche de l'axe central dudit rotor,
- tout en n'ayant qu'une fente sur la hauteur dudit rotor.

2. Séparateur centrifuge (S), comprenant un rotor, caractérisé en ce que

- ledit rotor est formé radialement d'une paroi spirale conique (48) servant de fente conique spirale qui sert de l'espace contenant le liquide,

- ladite paroi conique (48) comprenant un angle d'inclinaison (90° - $\alpha$) à l'axe central du rotor,
- ledit rotor comprenant un premier orifice (42) pour le liquide essentiellement au point le plus éloigné de l'axe central dudit rotor,
- et ensuite comprenant un deuxième orifice (43) pour le liquide essentiellement au point le plus proche de l'axe central dudit rotor,
- des fentes coniques coaxiales sont présentes, comportant un orifice communiquant qui s'étend sur la hauteur des fentes aux endroits où elles sont adjacentes.

3. Séparateur centrifuge (S) selon la revendication 1 ou 2 caractérisé en ce que l'angle d'inclinaison (90° - $\alpha$) est dans l'intervalle de 10° à 60°.

4. Séparateur centrifuge (S) selon la revendication 2, caractérisé en ce que le flux continu du fluide liquide dans l'espace de liquide est dirigé vers la gauche ou vers la droite, ou en alternant les deux successivement.

5. Séparateur centrifuge (S) selon l'une des revendications 1 à 4, caractérisé en ce que la limite supérieure de l'espace de fluide liquide est présente dans un plan.

6. Séparateur centrifuge (S) selon l'une des revendications 1 à 5, caractérisé en ce que la limite inférieure de l'espace de fluide liquide est présente dans un plan.

7. Séparateur centrifuge (S) selon l'une des revendications 1 à 6, caractérisé en ce que l'espace de liquide a une profondeur (d) de 2 à 10 mm.

8. Méthode de séparation de cellules animales et une solution aqueuse d'une suspension aqueuse contenant des cellules animales vivantes, caractérisée en ce que :

(A) on alimente le séparateur centrifuge rotatif (S) en une suspension aqueuse contenant des cellules animales vivantes par le premier orifice (32, 42, A) pour le fluide liquide, conformément à l'une des revendications 1 à 7, tout en soutirant en même temps la solution aqueuse séparée des cellules animales vivantes par le deuxième orifice (33, 43, B) pour le fluide liquide et en accumulant les cellules animales dans le bol du séparateur centrifuge (S) ;

(B) on apporte ensuite au séparateur centrifuge (S) un véhicule liquide non miscible à l'eau, ayant une densité supérieure à celle des cellules animales et à celle de la solution aqueuse, et n'inhibant pas la croissance des cellules animales, par le premier orifice (32, 42, A) pour le fluide liquide, en soutirant les cellules animales vivantes, conjointement avec la solution aqueuse restante, en les poussant vers l'extérieur par le véhicule liquide par le deuxième orifice (33, 43, B) pour le fluide liquide en obtenant ainsi les cellules animales vivantes, et

(C) on apporte de la solution fraîche ou du milieu épuisé supplémentaires par le deuxième orifice (33, 43, B) pour le fluide liquide au séparateur centrifuge (S), tout en soutirant le véhicule liquide par le premier orifice (32, 42, A) pour le fluide liquide, en vue de séparer le véhicule liquide.

9. Méthode selon la revendication 8, caractérisée en ce que la suspension aqueuse contenant les cellules animales vivantes est une suspension cultivée artificiellement.

10. Méthode selon la revendication 8, caractérisée en ce que la suspension aqueuse contenant les cellules animales vivantes est un sang de mammifère.

11. Méthode selon la revendication 8, 9 ou 10, caractérisée en ce que le véhicule liquide est un fluorocarbone.

12. Méthode selon l'une quelconque des revendications 8 à 11, caractérisée en ce que le séparateur centrifuge (S) est exploité dans les conditions suivantes :

(1) $\theta \leq 300$,
(2) $\overline{Z} \times \theta \leq 3 \times 10^4$,
(3) $Q/S \cdot \overline{Z} \leq 0,3$, et
(4) $5 \leq \overline{Z} \leq 2000$

dans lesquelles

θ est la durée de séjour moyenne (minutes) des cellules animales dans le séparateur centrifuge (S),

$\overline{Z}$ est un effet de centrifugation,

Q est la quantité (ml/mn) de fluide de culture en suspension apportée au séparateur centrifuge (S) par unité de temps, et

S est l'aire de sédimentation (en $cm^2$) sous l'effet de la force centrifuge.

**13.** Méthode de culture de cellules animales en suspension, caractérisée en ce que :

(a) on cultive des cellules animales vivantes en suspension dans un réacteur de culture (T1),

(b) on soutire une portion du fluide de culture en suspension contenant les cellules animales vivantes du réacteur de culture (T1),

(c) on alimente le séparateur centrifuge rotatif (S) en fluide de culture en suspension soutiré par le premier orifice (32, 42, A) pour le fluide liquide conformément à l'une quelconque des revendications 1 à 7, pendant un certain intervalle de temps, tout en soutirant le fluide de culture séparé des cellules vivantes, par le deuxième orifice (33, 43, B) pour le fluide liquide, et en accumulant les cellules animales dans le bol du séparateur centrifuge (S),

(d) ensuite on apporte un véhicule liquide non miscible à l'eau, ayant une densité supérieure à celle des cellules animales et à celles du fluide de culture et n'inhibant pas la croissance des cellules animales par le premier orifice (32, 42, A) pour le fluide liquide dans le séparateur centrifuge (S), et en soutirant les cellules animales vivantes en les poussant vers l'extérieur, conjointement avec le fluide de culture, par le deuxième orifice (33, 43, B) pour le fluide liquide,

(e) on retourne, au moins une partie des cellules animales soutirées, au réacteur de culture (T1) pour l'étape (a), et

(f) on apporte davantage de fluide de culture frais ou de fluide de culture soutiré à l'étape (c) par le deuxième orifice (33, 43, B) pour le fluide liquide dans le séparateur centrifuge (S), et on soutire le véhicule liquide par le premier orifice (32, 42, A) pour le fluide liquide afin de récupérer le véhicule liquide par le premier orifice (32, 42, A) par séparation.

**14.** Méthode selon la revendication 13, caractérisée en ce que l'on répète les étapes de (b) à (f).

**15.** Méthode selon la revendication 13 ou 14, caractérisée en ce que les cellules animales sont des cellules hybridomes.

**16.** Méthode selon l'une quelconque des revendications 13 à 15, caractérisée en ce que le véhicule liquide est un fluorocarbone.

**17.** Méthode selon l'une quelconque des revendications 13 à 16, caractérisée en ce que le séparateur centrifuge (S) est exploité dans les conditions suivantes :

(1) $\theta \leq 300$,

(2) $\overline{Z} \times \theta \leq 3 \times 10^4$,

(3) $Q/S \cdot \overline{Z} \leq 0{,}3$, et

(4) $5 \leq \overline{Z} \leq 2000$

dans lesquelles

θ est le temps de séjour moyen (minutes) des cellules animales dans le séparateur centrifuge (S),

$\overline{Z}$ est un effet de centrifugation,

Q est la quantité (ml/mn) de fluide de culture en suspension apportée au séparateur centrifuge (S) par unité de temps, et

S est l'aire de sédimentation (en $cm^2$) sous l'effet de la force centrifuge.

FIG. 1

FIG. 2

FIG. 4

FIG. 3(1)

FIG. 3(2)

FIG. 3(3)